# EUROPÄISCHE PATENTSCHRIFT

(11) **EP 2 740 497 B1**
(45) Veröffentlichungstag und Bekanntmachung des Hinweises auf die Patenterteilung: **06.12.2017**
(21) Anmeldenummer: 14157960.7
(22) Anmeldetag: 25.02.2010
(51) Int. Cl.: A61L 24/00, A61L 24/06

(54) **Medizinische Klebstoffzusammensetzung**
Medical adhesive compound
Composition de colle médicale

(30) Priorität: 26.02.2009 DE 102009012187
(43) Veröffentlichungstag der Anmeldung: 11.06.2014
(62) Teilanmeldung aus: 10001919.9
(73) Patentinhaber: Aesculap AG, 78532 Tuttlingen/Donau (DE)
(72) Erfinder: Siedle, Gabriel, 79117 Freiburg (DE)
(74) Vertreter: Patentanwälte Ruff, Wilhelm, Beier, Dauster & Partner mbB

(56) Entgegenhaltungen:
- EP-A1- 2 143 771
- DE-A1- 2 612 546
- DE-A1-102007 019 044
- US-A- 4 196 271
- US-A1- 2006 094 833

## Beschreibung

Die Erfindung betrifft eine medizinische Klebstoffzusammensetzung.

In der modernen Versorgungschirurgie kommt mittlerweile eine Vielzahl von Klebstoffzusammensetzungen zum Einsatz. Häufig basieren die Klebstoffzusammensetzungen auf Proteinen. Ein bekanntes Beispiel hierfür stellen Fibrinkleber dar. Fibrinkleber zeichnen sich durch eine gute Bioverträglichkeit sowie durch eine vollständige Resorbierbarkeit aus. Fibrinkleber werden beispielsweise zur Behandlung von blutenden Wunden eingesetzt. Nachteilig im Falle von Fibrinklebern ist jedoch ihre geringe Haftkraft. Hinzu kommt, dass Fibrinkleber sehr teuer sind und außerdem eine schlechte Lagerstabilität aufweisen. So müssen Fibrinkleber zur Lagerung bzw. Aufbewahrung eingefroren werden. Dies wiederum macht es erforderlich, dass die Fibrinkleber rechtzeitig vor ihrer Verwendung aufgetaut werden müssen, wodurch der Umgang mit diesen Klebern insgesamt umständlich und aufwendig wird. Darüber hinaus besteht bei Fibrinklebern ein gewisses Risiko für Infektionen.

Klebstoffzusammensetzungen auf der Basis von Cyanoacrylat-Monomeren besitzen dagegen eine hohe Haftkraft und sind relativ preiswert. Da es sich um synthetische Klebstoffzusammensetzungen handelt, besteht zudem keine Infektionsgefahr. Nachteilig hierbei ist jedoch, dass ausgehärtete Klebstoffzusammensetzungen auf der Basis von Cyanoacrylat-Monomeren häufig brüchig und spröde sind. Eine geringe Flexibilität der ausgehärteten Zusammensetzungen ist jedoch insbesondere im Hinblick auf sich rhythmisch ausdehnende Gewebe, wie beispielsweise Lungengewebe, oder pulsierende Gewebe, wie beispielsweise Blutgefäße, von Nachteil. So besteht die Gefahr, dass sich die ausgehärtete Klebstoffzusammensetzungen vom Gewebe ablösen und sich in das Gewebe einschneiden. Ein zuverlässiger Wundverschluss ist daher in solchen Fällen nicht gewährleistet.

Deswegen enthalten Klebstoffzusammensetzungen auf der Basis von Cyanoacrylat-Monomeren häufig Weichmacher. Diese erhöhen zwar die Flexibilität, insbesondere Biegefestigkeit, der ausgehärteten Klebstoffzusammensetzungen. Mit zunehmendem Weichmacheranteil sinkt jedoch die Haftfestigkeit der Klebstoffzusammensetzung, insbesondere auf ein Niveau, bei welchem ein zuverlässiger Wundverschluss ebenfalls nicht mehr gewährleistet ist. Die mangelnde Haftfestigkeit der ausgehärteten Klebstoffzusammensetzungen ist dabei auf die Entstehung von Ausblühungen im Verlaufe der Aushärtung der Klebstoffzusammensetzungen zurückzuführen. Infolge dieser Ausblühungen kommt es zur Ausbildung eines unregelmäßigen Klebstofffilmes, auf dessen Oberfläche sich häufig Kügelchen ausbilden und dessen Ränder sich in der Regel als Grat (unregelmäßig geformte Erhebungen an den Randzonen des Klebstofffilmes) ausbilden. Der vorstehend beschriebene Ausblüheffekt ist in der Literatur hinreichend beschrieben (The use of Indermil (n-butyl cyanoacrylate) in otorhinolaryngology and head and neck surgery. A preliminary report on the first 33 patients; P.R. Samuel et al., Journal of Laryngology & Otology; (1997); 111:536-540).

In der US 2003/ 0162857 A1 wird als Lösung dieses Problems die Verwendung von Photoinitiatoren vorgeschlagen. Photoinitiatoren sind jedoch für diverse medizinische Anwendungen, beispielsweise für Hautklebungen, ungeeignet, da ihr Einsatz aufwendig ist und Photoinitiatoren in der Regel toxisch sind.

Eine Klebstoffzusammensetzung, welche neben Cyanoacrylat-Monomeren und diversen Polymerisationsinitiatoren auch Fettsäuren als Stabilisatoren aufweisen kann, ist in der EP 2 143 771 A1 beschrieben.

Cyanoacrylat-Klebstoffzusammensetzungen, welche für topische Anwendungen u.a. höhere Fettsäuren als hautpflegende Aktivstoffe enthalten können, sind aus der DE 10 2007 019 044 A1 bekannt.

Aus der US 2006/0094833 A1 sind Cyanoacrylat-Klebstoffzusammensetzungen mit einem Carbonsäuregehalt von 5 ppm bis 5000 ppm bekannt. Gegenstand der US 4,196,271 sind carbonsäurehaltige Cyanoacrylat-Klebstoffzusammensetzungen mit einem Carbonsäuregehalt von 0.1 ppm bis 50000 ppm.

Die Erfindung stellt sich daher die Aufgabe, eine medizinische Klebstoffzusammensetzung bereitzustellen, welche eine nach ihrer Aushärtung auf biologischen Geweben verbesserte Haftfestigkeit aufweist, ohne dass hierdurch das übrige erwünschte Eigenschaftsprofil für medizinische Klebstoffzusammensetzungen, insbesondere im Hinblick auf Biegefestigkeit und Aushärtungszeit, in unerwünschter Weise beeinträchtigt wird.

Diese Aufgabe wird gelöst durch eine medizinische Klebstoffzusammen-setzung mit den Merkmalen des unabhängigen Anspruchs 1. Bevorzugte Ausführungsformen der Klebstoffzusammensetzung sind Gegenstand der abhängigen Ansprüche 2 bis 15. Der Wortlaut sämtlicher Ansprüche wird hiermit durch Bezugnahme zum Inhalt dieser Beschrei-bung gemacht.

Bei der erfindungsgemäßen Klebstoffzusammensetzung handelt es sich um eine medizinische Klebstoffzusammensetzung, vorzugsweise zur Verklebung von biologischen, insbesondere menschlichen und/oder tierischen, Geweben, umfassend Cyanoacrylat-Monomere, einen Weichmacher und eine Carbonsäure mit einer Kohlenstoffkette, die mehr als zwei Kohlenstoffatome aufweist.

Überraschender Weise wurde herausgefunden, dass bei Zusatz von Carbonsäuren, die eine Kohlenstoffkette mit mehr als zwei Kohlenstoffatomen besitzen, zu einer auf Cyanoacrylat-Monomeren basierenden Klebstoffzusammensetzung die Ausbildung der einleitend beschriebenen Ausblühungen während der Aushärtung der Klebstoffzusammensetzung verhindert bzw. signifikant verringert werden kann. Dadurch wird mit besonderem Vorteil die Haftfestigkeit der ausgehärteten Klebstoffzusammensetzung und damit auch die Sicherheit einer medizinischen, insbesondere chirurgischen, Verklebung erhöht. Gleichzeitig werden unter medizinischen Gesichtspunkten vorteilhafte Eigenschaften der Klebstoffzusammensetzung, insbesondere deren Flexibilität, vorzugsweise Biegefestigkeit, und Aushärtezeit, nicht negativ beeinflusst.

Bei der Carbonsäure handelt es sich vorzugsweise um eine Monocarbonsäure, insbesondere aliphatische Monocarbonsäure. Die Carbonsäure kann verzweigt oder unverzweigt sein. Vorzugsweise handelt es sich bei der Carbonsäure um eine unverzweigte Carbonsäure.

Die Carbonsäure ist vorzugsweise löslich in flüssigen Cyanoacrylat-Monomeren. Besonders bevorzugt ist die Carbonsäure löslich in einer flüssigen Klebstoffzusammensetzung, welche Cyanoacrylat-Monomere und einen Weichmacher enthält. Erfindungsgemäß ist es daher besonders bevorzugt, wenn die Klebstoffzusammensetzung als Lösung vorliegt. Lösungen besitzen allgemein den Vorteil, dass sie besser handhabbar und applizierbar sind. Beispielsweise lassen sich Lösungen besser injizieren oder versprühen als flüssige Dispersionen, insbesondere Suspensionen. Zudem haben Lösungen auch hinsichtlich ihrer Lagerfähigkeit Vorteile gegenüber flüssigen Dispersionen.

In einer bevorzugten Ausführungsform weist die Kohlenstoffkette der Carbonsäure zumindest 4 Kohlenstoffatome, insbesondere zumindest 8 Kohlenstoffatome, vorzugsweise zumindest 12 Kohlenstoffatome, auf. Die Kohlenstoffkette der Carbonsäure kann in einer weitergehenden Ausführungsform zwischen 4 und 24, insbesondere 8 und 22, vorzugsweise 14 und 18, Kohlenstoffatome aufweisen.

Des Weiteren handelt es sich bei der Carbonsäure vorzugsweise um eine bioverträgliche, insbesondere körperaffine, Carbonsäure.

In einer besonders bevorzugten Ausführungsform handelt es sich bei der Carbonsäure um eine Fettsäure. Die Carbonsäure kann eine niedere, mittlere und/oder höhere Fettsäure sein. Unter einer niederen Fettsäure soll im Sinne der Erfindung eine Fettsäure verstanden werden, welche eine Kohlenstoffkette mit 3 bis 7 Kohlenstoffatomen aufweist. Unter einer mittleren Fettsäure soll eine Fettsäure verstanden werden, welche eine Kohlenstoffkette zwischen 8 und 12 Kohlenstoffatome aufweist. Unter einer höheren Fettsäure soll eine Fettsäure verstanden werden, die eine Kohlenstoffkette mit mehr als 12 Kohlenstoffatome aufweist.

Als Fettsäuren kommen grundsätzlich gesättigte Fettsäuren und/oder ungesättigte Fettsäuren in Betracht. Bevorzugt ist die Carbonsäure eine Fettsäure aus der Gruppe Buttersäure, Valeriansäure, Capronsäure, Önanthsäure, Caprylsäure, Pelargonsäure, Caprinsäure, Undecylen-säure, Laurinsäure, Myristinsäure, Myristoleinsäure, Palmitinsäure, Palmitoleinsäure, Linolsäure, Margarinsäure, Stearinsäure, Petroselin-säure, Alpha-Linolensäure, Gamma-Linolensäure, Calendulasäure, Punicinsäure, Alpha-Eleostearinsäure, Arachinsäure, Gadoleinsäure, Icosensäure, Arachidonsäure, Timnodonsäure, Behensäure, Cetolein-säure, Erucasäure, Clupanodonsäure, Cervonsäure und Mischungen davon. Besonders bevorzugt handelt es sich bei der Carbonsäure um eine Fettsäure aus der Gruppe Myristinsäure, Palmitinsäure, Stearin-säure, Ölsäure, Linolsäure, Linolensäure und Mischungen davon.

Bei der Carbonsäure kann es sich in einer möglichen Ausführungsform auch um ein Carbonsäuresalz, insbesondere Fettsäuresalz, oder um eine Mischung von Carbonsäure- bzw. Fettsäuresalzen, beispielsweise um eine Mischung von Salzen der zuvor genannten Carbonsäuren bzw. Fettsäuren, handeln.

Die Klebstoffzusammensetzung weist einen Carbonsäureanteil ≥ 0,1 Gew.-%, insbesondere > 0,15 Gew.-%, bevorzugt > 0,2 Gew.% bezogen auf das Gesamtgewicht der Klebstoffzusammensetzung, auf. Die Klebstoffzusammensetzung kann insbesondere einen Carbonsäureanteil zwischen 0,1 und 2 Gew.%, vorzugsweise 0,1 und 1 Gew.-%, bezogen auf das Gesamtgewicht der Klebstoffzusammensetzung, aufweisen. Bevorzugt weist die Klebstoffzusammensetzung einen Carbonsäureanteil zwischen 0,2 und 2 Gew.-%, insbesondere 0,2 und 1 Gew.-%, auf, bezogen auf das Gesamtgewicht der Klebstoffzusammensetzung.

Der erfindungsgemäß vorgesehene Weichmacher ist vorzugsweise inert gegenüber Cyanoacrylat-Monomeren. Auf diese Weise kann eine vorzeitige Polymerisation der Cyanoacrylat-Monomere und damit eine vorzeitige Aushärtung der erfindungsgemäßen Klebstoffzusammensetzung verhindert werden. Besonders bevorzugt handelt es sich bei dem Weichmacher zudem um einen in Cyanoacrylat-Monomeren löslichen Weichmacher. Die Klebstoffzusammensetzung weist gemäß einer weiteren Ausführungsform einen Weichmacheranteil zwischen 5 und 50 Gew.-%, bevorzugt 5 und 30 Gew.-%, auf, bezogen auf das Gesamtgewicht der Klebstoffzusammensetzung.

Bevorzugt ist der Weichmacher aus der Gruppe Glycerinester, Polyethylenglykol, Glykolester, Polyethylenglykolester, Milchsäureester, Valeriansäureester, Buttersäureester, Oxalsäureester, Äpfelsäureester, Weinsäureester, Glutarsäureester, Bernsteinsäureester, Zitronensäure- bzw. Citratester, Maleinsäureester, Fumarsäureester, Malonsäureester, Acetessigsäureester, Zitronsäure- bzw. Citratester, Laevulinsäureester, Glycerinacetatester, Propiolsäureester, Essigsäureester, Glykolsäureester, Sebacinsäureester, Phthalsäureester, Fettsäureester, Phosphatester, Celluloseester, Zuckeralkohole, Ethylenglykole, flüssige Polyethylenglykole, Propylen-1, 2-Glykole, flüssige Polypropylen-1,2-Glykole, Propylen-1,3-Glykole, flüssige Polypropylen-1,3-Glykole, Polyethylenglykolether und Mischungen davon ausgewählt.

In einer weitergehenden Ausführungsform ist der Weichmacher aus der Gruppe Glycerintriacetat, Glycerintripropionat, Triglycerinpentaacetat, Polygly-cerinacetat, Glycerintriopropionat, Glycerintributyrat, Glycerintrihexanoat, Diethylenglykol-diacetat, 3-Hydroxyvaleriansäureethylester, Milchsäurebutylester, Milch-säureisobutylester, 3-Hydroxybuttersäureethylester, Oxalsäurediethyl-ester, Mesoxalsäurediethylester, Äpfelsäuredimethylester, Äpfelsäuredi-isopropylester, Weinsäurediethylester, Weinsäuredipropylester, Weinsäurediisopropylester, Tributylacetylcitrat, Glutarsäuredimethylester, Bernsteinsäuredime-thylester, Bernsteinsäurediethylester, Maleinsäurediethylester, Fumar-säurediethylester, Malonsäurediethylester, Glycerindiacetat, Glycerindipropionat, Glycerintriisobutyrat, Glycerindiisobutyrat, Glycidylbutyrat, Acetessigsäurebutylester, Levolinsäureethylester, 3-Hydroxyglutarsäure-dimethylester, Glycerinacetatdipropionat, Glycerindiacetatbutyrat, Pro-piolsäurebutylester, Propylenglykoldiacetat, Propylenglykoldibutyrat, Diethylenglykoldibutyrat, Trimethylolethantriacetat, Trimethylolethan-tributyrat, Neopentylalkoholdibutyrat, Metoxyessigsäurepentylester, Dimethoxyessigsäurebutylester, Glykolsäurebutylester, Tricaproin, Trivalerin, Tricaprin, Dibutylphthalat, Dioctylphthalat, Diallylphthalat, Butylbenzylphthalat, Isobutylmyristat, Ethylmyristat, Ethylstearat, Iso-propylmyristat, Butylstearat, Methylsebacat, Ethylsebacat, Triethyl-phosphat, Tri(2-ethylhexyl)-phosphat, Tri(p-cresyl)-phosphat, Dibutylphosphat, Diphenyl-cresyl-phosphat, Butylbenzylphosphat, Ethylcellu-Iose, Polyethylenglykoldimethylether, Propylen-1,2-Glykol, Propylen-1,3-Glykol, 2-Ethyl-2-Hydroxymethyl-1,3-Propandiol und Mischungen davon ausgewählt. Im Übrigen handelt es sich im Falle von Fettsäureestern bevorzugt um die Ester der in der vorliegenden Beschreibung genannten Fettsäuren.

Gemäß einer besonders bevorzugten Ausführungsform handelt es sich bei dem Weichmacher um einen Zitronsäureester bzw. Citratester, insbesondere um einen Teil- oder Vollester davon. Ein bevorzugter Zitronensäureester bzw. Citratester ist Tributylacetylcitrat.

Erfindungsgemäß bevorzugt sind Weichmacher, welche an sich bereits geringere Ausblühungen verursachen. Beispielhaft werden in diesem Zusammenhang Glycerintriacetat und/oder Glycerintrihexanoat genannt.

Die Klebstoffzusammensetzung weist in einer weiteren Ausführungsform bei einem Carbonsäureanteil zwischen 0,1 und 1 Gew.-% und bei einem Weichmacheranteil zwischen 5 und 35 Gew.-%, jeweils bezogen auf das Gesamtgewicht der Klebstoffzusammensetzung, und bei einer Temperatur von 25 °C eine Viskosität zwischen 3 und 100 mPas, insbesondere 3 und 30 mPas, auf.

Erfindungsgemäß können die Cyanoacrylat-Monomere aus der Gruppe Alkyl-Cyanoacrylat-Monomere, Alkoxyalkyl-Cyanoacrylat-Monomere, Alkylester-Cyanoacrylat-Monomere und Mischungen davon ausgewählt sein. Die Alkylgruppen der vorstehend aufgeführten Cyanoacrylat-Monomere können dabei eine Kohlenstoffkette mit 1 bis 20, insbesondere 2 bis 10, vorzugsweise 4 bis 8, Kohlenstoffatomen aufweisen. Bevorzugt sind die Cyanoacrylat-Monomere aus der Gruppe n-Butyl-Cyanoacrylat-Monomere, Iso-Butyl-Cyanoacrylat-Monomere, Pentyl-Cyanoacrylat-Monomere, Hexyl-Cyanoacrylat-Monomere, Octyl-Cyanoacrylat-Monomere, Allyl-Cyanoacrylat-Monomere, Methallyl-Cyanoacrylat-Monomere, Crotyl-Cyanoacrylat-Monomere, Propargyl-Cyanoacrylat-Monomere, Methoxyethyl-Cyanoacrylat-Monomere, Ethoxyethyl-Cyanoacrylat-Monomere, Methoxypropyl-Cyanoacrylat-Monomere, Methoxybutyl-Cyanoacrylat-Monomere, Butyllactoyl-Cyanoacrylat-Monomere und Mischungen davon ausgewählt. Besonders bevorzugt handelt es sich bei den Cyanoacrylat-Monomeren um n-Butyl-Cyanoacrylat-Monomere, Octyl-Cyanoacrylat-Monomere, Ethoxyethyl-Cyanoacrylat-Monomere, Methoxypropyl-Cyanoacrylat-Monomere, Methoxybutylcyanoacrylat-Monomere, Butyllactoyl-Cyanoacrylat-Monomere und/oder Mischungen davon. n-Butyl-Cyanoacrylat-Monomere sind vor allem wegen ihrer hohen Haftkraft bevorzugt. Beispielsweise ist eine entsprechende Klebstoffzusammensetzung unter der Bezeichnung Histoacryl® kommerziell erhältlich. Ethoxyethyl-Cyanoacrylat-Monomere und/oder Methoxybutyl-Cyanoacrylat-Monomere sind zum einen wegen ihrer grundsätzlich geringeren Neigung zur Ausbildung von Ausblühungen während des Aushärtevorganges bevorzugt. Zum anderen sind sie auch wegen ihrer besonders schnellen Resorbierbarkeit von Vorteil.

In einer weiteren Ausführungsform weist die Klebstoffzusammensetzung eine Topf- bzw. Gelzeit (Aushärtezeit) t < 90 Sekunden, vorzugsweise < 60 Sekunden, auf. Als Topf- bzw. Gelzeit wird die Zeit definiert, ab welcher die viskoelastischen Eigenschaften die fließfähigen Eigenschaften einer zwei- oder mehrkomponentigen Mischung übersteigen. Durch die kurze Topf- bzw. Gelzeit kommt es mit besonderem Vorteil zu einer raschen Aushärtung der erfindungsgemäßen Klebstoffzusammensetzung nach Applikation auf ein zu versorgendes Gewebeareal. Dadurch können sich die klebenden bzw. verklebenden Eigenschaften der Klebstoffzusammensetzung in relativ kurzer Zeit entfalten, ohne dass die Klebstoffzusammensetzung aus dem zu versorgenden Gewebeareal weggespült wird.

In einer weiteren Ausführungsform ist die Klebstoffzusammensetzung bei Zusatz eines Aktivators bzw. Initiators, vorzugsweise Nukleophils, zu einer flexiblen, insbesondere biegefesten, Klebstoffverbindung, in der Regel in Form eines Klebstofffilms oder - überzugs, aushärtbar.

Erfindungsgemäß kann es weiterhin vorgesehen sein, dass die Klebstoffzusammensetzung Additive, insbesondere aus der Gruppe Stabilisatoren, Radikalfänger, Formaldehydfänger, antimikrobielle Wirkstoffe, desinfizierende Wirkstoffe, wundheilungsfördernde Wirkstoffe, blutgerinnungsfördernde Wirkstoffe, schmerzstillende Wirkstoffe, geruchsbekämpfende Wirkstoffe und Kombinationen davon, enthält. Als Stabilisatoren kommen insbesondere Säuren und/oder Säureanhydride, beispielsweise Schwefeldioxid, Schwefelsäure, Essigsäure, Phosphorsäure, Borsäurederivate und/oder Sulfonsäuren, in Betracht. Beispiele für geeignete Radikalfänger sind aus der Gruppe Hydrochinon, t-Butyl-Hydroxyanisol, 3,5-Di-tert.-Butyl-4-Hydroxytoluol und Kombinationen davon ausgewählt. Als blutgerinnungsfördernde Wirkstoffe kommen beispielsweise Calciumsalze in Betracht. Beispiele für geeignete Formaldehydfänger sind Sulfid, Bisulfid, Ammoniumsulfid und/oder Harnstoffsalze. Als wundheilungsfördernde Wirkstoffe können beispielsweise Hyaluronsäure und/oder Zinkverbindungen, insbesondere Zinksalze, beispielsweise Zinkoxid und/oder Zinkhyaluronat, verwendet werden.

Wie bereits erwähnt, eignet sich die erfindungsgemäße Klebstoffzusammensetzung bevorzugt zur Verklebung von biologischen, insbesondere menschlichen und/oder tierischen, Geweben. Vorzugsweise handelt es sich bei der Klebstoffzusammensetzung daher um eine chirurgische Klebstoffzusammensetzung, insbesondere zur Verklebung von menschlichen und/oder tierischen Hart- und/ oder Weichgeweben. Als zu verklebende Gewebe kommen insbesondere Knochen, Muskeln, Faszien, Fett, Haut, Organe, Nerven, Blutgefäße, Bindegewebe, Sehnen oder Bänder in Betracht. Erfindungsgemäß eignet sich die Klebstoffzusammensetzung insbesondere zur Verwendung als flüssiges Hämostyptikum, vorzugsweise zur Versorgung von parenchymatösen Blutungen. In einer weitergehenden Ausführungsform eignet sich die Klebstoffzusammensetzung zum Verschluss von Flüssigkeits- und/oder Luftleckagen im menschlichen und/oder tierischen Körper, insbesondere zum Verschluss von Lungenleckagen, Blasenleckagen, Harnleiterleckagen, Herzbeutelleckagen und/ oder cereprospinalen Flüssigkeitsleckagen. Ein weiteres Anwendungsgebiet betrifft den Verschluss von Anastomosen, insbesondere Darm- oder Gefäßanastomosen. Allgemein lassen sich mit der Klebstoffzusammensetzung oberflächliche Wunden, insbesondere Schürfwunden, und/oder innere Wunden, beispielsweise Organblutungen, verschließen. Besonders bevorzugt wird die erfindungsgemäße Zusammensetzung zur keimdichten Versiegelung von Hautwunden und Inzisionen verwendet. Ein weiteres Anwendungsgebiet der Klebstoffzusammen-setzung betrifft schließlich seine Verwendung als Fixierungsmittel für Implantate. Bei den zu fixierenden Implantaten kann es sich dabei um Herniennetze, Harninkontinenznetze, Prolapsnetze, Dura Mater-Ersatzmaterialien und/oder Wundverbände handeln.

Weitere Merkmale der Erfindung ergeben sich durch die nachfolgende Beschreibung von bevorzugten Ausführungsformen in Form von Beispielen in Verbindung mit den Unteransprüchen. Hierbei können einzelne Merkmale der Erfindung alleine oder in Kombination miteinander verwirklicht sein. Die beschriebenen Ausführungsformen dienen dabei lediglich zur Erläuterung und zum besseren Verständnis der Erfindung und sind in keiner Weise einschränkend zu verstehen.

### Beispiel 1: Untersuchung des Einflusses eines Carbonsäurezusatzes auf die Entstehung von Ausblühungen

Zu einer Klebstoffzusammensetzung, welche einen Anteil an n-Butyl-Cyanoacrylat von 77,5 Gew.% und einen Anteil an Glycerintriacetat (Triacin) von 22,5 Gew.-% enthielt (jeweils bezogen auf das Gesamtgewicht der Klebstoffzusammensetzung), wurden in verschiedenen Ansätzen die in unten stehender Tabelle 1 aufgeführten Fettsäuren in solchen Mengen hinzugegeben, dass die hieraus resultierenden Klebstoffzusammensetzungen einen Anteil der jeweiligen Fettsäuren aufwiesen, wie ebenfalls in der Tabelle 1 dargestellt. Danach wurden die Klebstoffzusammensetzungen mit einem mit 0,1 Gew.-%iger Histidin-Lösung als Initiatorlösung durchtränkten OP-Tuch in Kontakt gebracht, um die Aushärtung der Klebstoffzusammensetzungen zu initiieren. Die hierbei erhaltenen Ergebnisse im Hinblick auf die mögliche Entstehung von Ausblühungen ist in der Tabelle 1 dargestellt.

**Tabelle 1**

| Anteil (Gew.-%) | 0,05 % | 0,1 % | 0,5 % | 1,0 % |
|---|---|---|---|---|
| Myristinsäure | starke Ausblühungen | wenig Ausblühungen | keine Ausblühungen | keine Ausblühungen |
| Palmitinsäure | starke Ausblühungen | wenig Ausblühungen | keine Ausblühungen | keine Ausblühungen |
| Stearinsäure | starke Ausblühungen | wenig Ausblühungen | wenig Ausblühungen | keine Ausblühungen |
| Ölsäure | starke Ausblühungen | wenig Ausblühungen | wenig Ausblühungen | keine Ausblühungen |

Die in Tabelle 1 dargestellten Ergebnisse zeigen, dass ausgehärtete Klebstoffzusammensetzungen, welche einen Carbonsäureanteil > 0,1 Gew.-%, bezogen auf das Gesamtgewicht der jeweiligen Klebstoffzusammensetzung, aufwiesen, zu keinen bzw. keinen nennenswerten Ausblühungen führten.

### Beispiel 2: Untersuchung der Biegefestigkeit von erfindungsgemäßen Klebstoffzusammensetzungen

Klebstoffzusammensetzungen auf der Basis von n-Butyl-Cyanoacrylat mit Weichmacheranteilen, wie sie in unten stehender Tabelle 2 wiedergegeben sind, sowie einem Myristinsäureanteil von 0,03 Gew.-% wurden jeweils auf ein OP-Tuch, welches zuvor mit einer 0,1 Gew.-%igen Histidinlösung als Initiatorlösung getränkt wurde, aufgetragen. Man ließ die Klebstoffzusammensetzungen danach für 24 Stunden aushärten. Im Anschluss daran wurden die ausgehärteten Proben auf ihre Biegefestigkeit hin überprüft. Die hierbei erhaltenen Ergebnisse sind in Tabelle 2 dargestellt.

**Tabelle 2**

| Anteil (Gew.-%) | 10 % | 20 % | 30 % | 40 % |
|---|---|---|---|---|
| Glycerintriacetat | bricht | bricht nicht | bricht nicht | bricht nicht |
| Glycerintributyrat | bricht | bricht | bricht nicht | bricht nicht |
| Glycerintrihexanoat | bricht | bricht | bricht nicht | bricht nicht |
| Tributylacetylcitrat | bricht | bricht nicht | bricht nicht | bricht nicht |
| Ethylstearat | bricht | bricht | bricht | bricht nicht |
| Diethylsebacat | bricht | bricht | bricht | bricht |

### Beispiel 3: Untersuchung der Haftkraft von erfindungsgemäßen Klebstoffzusammensetzungen

Zwei Schweinehautstücke von 2x7 cm wurden mit der Schmalseite aneinandergelegt. Danach wurden in getrennten Versuchen jeweils 20 µl von Klebstoffzusammensetzungen, wie sie in unten stehender Tabelle 3 aufgeführt sind, auf einer Nahtstelle verteilt. Nach einer Aushärtezeit von 15 Minuten wurde mit einer Zugprüfmaschine die Reißfestigkeit der Prüfkörper bestimmt. Die hierbei erhaltenen Ergebnisse sind in Tabelle 3 dargestellt.

**Tabelle 3**

| Zusätze zu n-Butyl-Cyanoacrylat | max. Reißkraft der Klebung |
|---|---|
| Glycerintriacetat 10 %, Myristinsäure 0,15 % | 20 N |
| Glycerintriacetat 20 %, Myristinsäure 0,15 % | 20 N |
| Glycerintriacetat 30 %, Myristinsäure 0,15 % | 10 N |

In einem Tierversuch wurden an Ratten an jeder Flanke eine Inzision von ca. 2 cm durchgeführt. Die resultierenden Wunden wurden jeweils mit Klebstoffzusammensetzungen, wie sie in unten stehender Tabelle 4 aufgeführt sind, verschlossen. Die Sicherheit der Klebung wurde mittels eines Skin Test Systems der Firma SRLI Technologies überprüft. Die hierbei erhaltenen Ergebnisse sind in Tabelle 4 dargestellt.

**Tabelle 4**

| Mischung | Bruch bei max. Unterdruck |
|---|---|
| NBCA + Glycerintriacetat 20 % | 152 mbar |
| NBCA + Glycerintriacetat 20 %, Myristinsäure 0,15 % | 308 mbar |

### NCBA = n-Butyl-Cyanoacrylat-Monomere

Aus Tabelle 4 geht hervor, dass der Zusatz von Myristinsäure im Vergleich zu einer herkömmlichen Klebstoffzusammensetzung zu einer Verdopplung der Belastbarkeit der Klebung führt. Dieses Ergebnis zeigt daher, dass erfindungsgemäße Klebstoffzusammensetzungen eine gegenüber herkömmlichen Klebstoffzusammensetzungen deutlich erhöhte Wundverschlusssicherheit gewährleisten können.

### Beispiel 4: Lagerstabilität

Eine Klebstoffzusammensetzung mit den Anteilen n-Butyl-Cyanoacrylat 77,5 Gew.-%, Glycerintriacetat (Triacetin) 22,3 Gew.-% und Myristinsäure 0,15 Gew.-% wurde zu je 0,6 ml in Kunststoffampullen abgefüllt. Die Ampullen wurden anschließend jeweils in einen Alubeutel verpackt und für 28 Tage bei 60 °C gelagert. Alle Proben waren danach noch flüssig. Eine vorzeitige Aushärtung der Klebstoffzusammensetzungen fand somit nicht statt.

## Patentansprüche

1. Medizinische Klebstoffzusammensetzung zur Verwendung zur Verklebung von biologischen, insbesondere menschlichen und/oder tierischen, Geweben, umfassend Cyanoacrylat-Monomere, einen Weichmacher und eine Carbonsäure mit einer Kohlenstoffkette, die mehr als zwei Kohlenstoffatome aufweist, wobei die Klebstoffzusammensetzung einen Carbonsäureanteil ≥ 0,1 Gew.-%, bezogen auf das Gesamtgewicht der Klebstoffzusammensetzung, aufweist.

2. Medizinische Klebstoffzusammensetzung nach Anspruch 1, **dadurch gekennzeichnet, dass** die Kohlenstoffkette der Carbonsäure zumindest 4 Kohlenstoffatome, insbesondere zumindest 8 Kohlenstoffatome, vorzugsweise mehr als 12 Kohlenstoffatome, aufweist.

3. Medizinische Klebstoffzusammensetzung nach Anspruch 1 oder 2, **dadurch gekennzeichnet, dass** es sich bei der Carbonsäure um eine Fettsäure handelt.

4. Medizinische Klebstoffzusammensetzung nach einem der vorhergehenden Ansprüche, **dadurch gekennzeichnet, dass** es sich bei der Carbonsäure um eine Fettsäure aus der Gruppe Myristinsäure, Palmitinsäure, Stearinsäure, Ölsäure, Linolsäure, Linolensäure und Mischungen davon handelt.

5. Medizinische Klebstoffzusammensetzung nach einem der vorhergehenden Ansprüche, **dadurch gekennzeichnet, dass** die Klebstoffzusammensetzung einen Carbonsäureanteil zwischen 0,1 und 2 Gew.-%, vorzugsweise zwischen 0,1 und 1 Gew.-%, bezogen auf das Gesamtgewicht der Klebstoffzusammensetzung, aufweist.

6. Medizinische Klebstoffzusammensetzung nach einem der vorhergehenden Ansprüche, **dadurch gekennzeichnet, dass** sie einen Weichmacheranteil zwischen 5 und 50 Gew.-%, bevorzugt 5 und 30 Gew.-%, aufweist, bezogen auf das Gesamtgewicht der Klebstoffzusammensetzung.

7. Medizinische Klebstoffzusammensetzung nach einem der vorhergehenden Ansprüche, **dadurch gekennzeichnet, dass** der Weichmacher aus der Gruppe Glycerinester, Polyethylenglykol, Glykolester, Polyethylenglykolester, Milchsäureester, Valerian-säureester, Buttersäureester, Oxalsäureester, Äpfelsäureester, Weinsäureester, Zitronsäure- bzw. Citratester, Glutarsäureester, Bernsteinsäureester, Maleinsäureester, Fumarsäureester, Malonsäureester Acetessigsäureester, Laevulinsäureester, Glycerinacetatester Propiolsäureester, Essigsäureester, Glykolsäureester, Sebachsäureester Phtalsäureester Fettsäureester, Phosphatester Celluloseester, Zuckeralkohole, Ethylenglykole, flüssige Polyethylenglykole, Propylen-1 2-Glykole, flüssige Polypropylen-1 ,2-Glykole, Propylen-1,3-Glykole, flüssige Polypropylen-1,3-Glykole, Polyethylenglykolether und Mischungen davon ausgewählt ist.

8. Medizinische Klebstoffzusammensetzung nach einem der vorhergehenden Ansprüche, **dadurch gekennzeichnet, dass** die Cyanoacrylat-Monomere aus der Gruppe Alkyl-Cyanoacrylat-Monomere, Alkoxyalkyl-Cyanoacrylat-Monomere, Alkylester-Cyanoacrylat-Monomere und Mischungen davon ausgewählt sind.

9. Medizinische Klebstoffzusammensetzung nach einem der vorhergehenden Ansprüche, **dadurch gekennzeichnet, dass** es sich bei den Cyanoacrylat-Monomeren um n-Butyl-Cyanoacrylat-Monomere, n-Octyl-Cyanoacrylat-Monomere, Ethoxyethyl-Cyano-acrylat-Monomere, Methoxypropyl-Cyanoacrylat-Monomere, Me-thoxybutyl-Cyanoacrylat-Monomere, Butyllactoyl-Cyanoacrylat-Monomere und/ oder Mischungen davon handelt.

10. Medizinische Klebstoffzusammensetzung nach einem der vorhergehenden Ansprüche, **dadurch gekennzeichnet, dass** es sich bei der Klebstoffzusammensetzung um eine chirurgische Klebstoffzusammensetzung, insbesondere zur Verwendung zur Verklebung von menschlichen und/oder tierischen Hart- und/oder Weichgeweben, handelt.

11. Medizinische Klebstoffzusammensetzung nach einem der vorhergehenden Ansprüche zur Verwendung als flüssiges Hämostyptikum, insbesondere zur Versorgung von parenchymatösen Blutungen.

12. Medizinische Klebstoffzusammensetzung nach einem der vorhergehenden Ansprüche zum Verschluss von Flüssigkeits- und/oder Luftleckagen im menschlichen und/oder tierischen Körper, insbesondere Lungenleckagen, Blasenleckagen, Harnleiterleckagen, Herzbeutelleckagen und/oder cerebrospinalen Flüssigkeitsleckagen.

13. Medizinische Klebstoffzusammensetzung nach einem der vorhergehenden Ansprüche zum Verschluss von Anastomosen, insbesondere Darm- oder Gefäßanastomosen.

14. Medizinische Klebstoffzusammensetzung nach einem der vorhergehenden Ansprüche zur keimdichten Versiegelung von Hautwunden und Inzisionen.

15. Medizinische Klebstoffzusammensetzung nach einem der vorhergehenden Ansprüche als Fixierungsmittel für Implantate.

## Claims

1. Medical adhesive composition for use for the bonding of biological tissues, more particularly human and/or animal tissues, comprising cyanoacrylate monomers, a plasticizer and a carboxylic acid having a carbon chain including more than two carbon atoms, wherein the adhesive composition has a carboxylic acid fraction ≥ 0.1 % by weight, based on the total weight of the adhesive composition.

2. Medical adhesive composition according to Claim 1, **characterized in that** the carbon chain of the carboxylic acid has at least 4 carbon atoms, in particular at least 8 carbon atoms, preferably more than 12 carbon atoms.

3. Medical adhesive composition according to Claim 1 or 2, **characterized in that** the carboxylic acid is a fatty acid.

4. Medical adhesive composition according to any of the preceding claims, **characterized in that** the carboxylic acid is a fatty acid from the group myristic acid, palmitic acid, stearic acid, oleic acid, linoleic acid, linolenic acid and mixtures thereof.

5. Medical adhesive composition according to any of the preceding claims, **characterized in that** the adhesive composition has a carboxylic acid fraction between 0.1 % and 2% by weight, preferably between 0.1 % and 1% by weight, based on the total weight of the adhesive composition.

6. Medical adhesive composition according to any of the preceding claims, **characterized in that** it has a plasticizer fraction of between 5% and 50% by weight, preferably 5% and 30% by weight, based on the total weight of the adhesive composition.

7. Medical adhesive composition according to any of the preceding claims, **characterized in that** the plasticizer is selected from the group glycerol esters, polyethylene glycol, glycol esters, polyethylene glycol esters, lactic esters, valeric esters, butyric esters, oxalic esters, malic esters, tartaric esters, citric esters and citrate esters, glutaric esters, succinic esters, maleic esters, fumaric esters, malonic esters, acetoacetic esters, laevulinic esters, glycerol acetate esters, propiolic esters, acetic esters, glycolic esters, sebacic esters, phthalate acid esters, fatty acid esters, phosphate esters, cellulose esters, sugar alcohols, ethylene glycols, liquid polyethylene glycols, propylene 1,2-glycols, liquid polypropylene 1,2-glycols, propylene 1,3-glycols, liquid polypropylene 1,3-glycols, polyethylene glycol ethers and mixtures thereof.

8. Medical adhesive composition according to any of the preceding claims, **characterized in that** the cyanoacrylate monomers are selected from the group alkyl cyanoacrylate monomers, alkoxyalkyl cyanoacrylate monomers, alkyl ester cyanoacrylate monomers and mixtures thereof.

9. Medical adhesive composition according to any of the preceding claims, **characterized in that** the cyanoacrylate monomers are n-butyl cyanoacrylate monomers, n-octyl cyanoacrylate monomers, ethoxyethyl cyanoacrylate monomers, methoxypropyl cyanoacrylate monomers, methoxybutyl cyanoacrylate monomers, butyllactoyl cyanoacrylate monomers and/or mixtures thereof.

10. Medical adhesive composition according to any of the preceding claims, **characterized in that** the adhesive composition is a surgical adhesive composition, in particular for use for the bonding of human and/or animal hard and/or soft tissues.

11. Medical adhesive composition according to any of the preceding claims for use as a liquid haemostyptic, more particularly for management of parenchymatous haemorrhages.

12. Medical adhesive composition according to any of the preceding claims for closure of leaks of fluid and/or air in the human and/or animal body, more particularly lung leaks, bladder leaks, ureter leaks, pericardium leaks and/or cerebrospinal fluid leaks.

13. Medical adhesive composition according to any of the preceding claims for closure of anastomoses, more particularly intestinal or vascular anastomoses.

14. Medical adhesive composition according to any of the preceding claims for germproof sealing of skin wounds and incisions.

15. Medical adhesive composition according to any of the preceding claims as a fixative for implants.

## Revendications

1. Composition adhésive médicale pour utilisation pour le collage de tissus biologiques, notamment humains et/ou animaux, comprenant des monomères de cyanoacrylate, un plastifiant et un acide carboxylique à chaîne carbonée comprenant plus de deux atomes de carbone, la composition adhésive présentant une proportion d'acide carboxylique ≥ 0.1 % en poids, par rapport au poids total de la composition adhésive.

2. Composition adhésive médicale selon la revendication 1, **caractérisée en ce que** la chaîne carbonée de l'acide carboxylique comprend au moins 4 atomes de carbone, en particulier au moins 8 atomes de carbone, de préférence plus de 12 atomes de carbone.

3. Composition adhésive médicale selon la revendication 1 ou 2, **caractérisée en ce que** l'acide carboxylique est un acide gras.

4. Composition adhésive médicale selon l'une quelconque des revendications précédentes, **caractérisée en ce que** l'acide carboxylique est un acide gras du groupe constitué par l'acide myristique, l'acide palmitique, l'acide stéarique, l'acide oléique, l'acide linoléique, l'acide linolénique et leurs mélanges.

5. Composition adhésive médicale selon l'une quelconque des revendications précédentes, **caractérisée en ce que** la composition adhésive présente une proportion d'acide carboxylique comprise entre 0,1 et 2 % en poids, de préférence entre 0,1 et 1 % en poids, par rapport au poids total de la composition adhésive.

6. Composition adhésive médicale selon l'une quelconque des revendications précédentes, **caractérisée en ce qu'**elle présente une proportion de plastifiant comprise entre 5 et 50 % en poids, de préférence entre 5 et 30 % en poids, par rapport au poids total de la composition adhésive.

7. Composition adhésive médicale selon l'une quelconque des revendications précédentes, **caractérisée en ce que** le plastifiant est choisi dans le groupe constitué par les esters de glycérine, le polyéthylène glycol, les esters de glycol, les esters de polyéthylène glycol, les esters de l'acide lactique, les esters de l'acide valérianique, les esters de l'acide butyrique, les esters de l'acide oxalique, les esters de l'acide malique, les esters de l'acide tartrique, les esters de l'acide citrique et de citrate, les esters de l'acide glutarique, les esters de l'acide succinique, les esters de l'acide maléique, les esters de l'acide fumarique, les esters de l'acide malonique, les esters de l'acide acétoacétique, les esters de l'acide lévulinique, les esters d'acétate de glycérine, les esters de l'acide propiolique, les esters de l'acide acétique, les esters de l'acide glycolique, les esters de l'acide sébacique, les esters de l'acide phtalique, les esters d'acides gras, les esters de phosphate, les esters de cellulose, les polyols, les éthylène glycols, les polyéthylène glycols liquides, les propylène-1,2-glycols, les polypropylène-1,2-glycols liquides, les propylène-1,3-glycols, les polypropylène-1,3-glycols liquides, les éthers de polyéthylène glycol et leurs mélanges.

8. Composition adhésive médicale selon l'une quelconque des revendications précédentes, **caractérisée en ce que** les monomères de cyanoacrylate sont choisis dans le groupe constitué par les monomères de cyanoacrylate d'alkyle, les monomères de cyanoacrylate d'alcoxyalkyle, les monomères de cyanoacrylate d'esters alkyliques et leurs mélanges.

9. Composition adhésive médicale selon l'une quelconque des revendications précédentes, **caractérisée en ce que** les monomères de cyanoacrylate sont des monomères de cyanoacrylate de n-butyle, des monomères de cyanoacrylate de n-octyle, des monomères de cyanoacrylate d'éthoxyéthyle, des monomères de cyanoacrylate de méthoxypropyle, des monomères de cyanoacrylate de méthoxybutyle, des monomères de cyanoacrylate de butyllactoyle et/ou leurs mélanges.

10. Composition adhésive médicale selon l'une quelconque des revendications précédentes, **caractérisée en ce que** la composition adhésive est une composition adhésive chirurgicale notamment destinée à une utilisation pour le collage de tissus durs et/ou mous humains et/ou animaux.

11. Composition adhésive médicale selon l'une quelconque des revendications précédentes destinée à une utilisation en tant qu'hémostyptique liquide, notamment pour le soin des hémorragies parenchymateuses.

12. Composition adhésive médicale selon l'une quelconque des revendications précédentes pour la fermeture de fuites de liquide et/ou d'air dans le corps humain et/ou animal, notamment de fuites pulmonaires, de fuites vésicales, de fuites urétérales, de fuites péricardiales et/ou de fluides de liquide cérébro-spinal.

13. Composition adhésive médicale selon l'une quelconque des revendications précédentes pour la fermeture d'anastomoses, notamment d'anastomoses intestinales ou vasculaires.

14. Composition adhésive médicale selon l'une quelconque des revendications précédentes pour l'étanchéification hermétique aux germes de lésions cutanées et d'incisions.

15. Composition adhésive médicale selon l'une quelconque des revendications précédentes en tant qu'agent de fixation pour implants.
